# EUROPEAN PATENT SPECIFICATION

(11) **EP 3 658 669 B1**
(45) Date of publication and mention of the grant of the patent: **05.06.2024**
(21) Application number: 18742517.8
(22) Date of filing: 25.07.2018
(51) Int. Cl.: C12N 9/04, C12Q 1/00, C12Q 1/32

(54) **MUTATED CELLOBIOSE DEHYDROGENASE WITH MODIFIED SUBSTRATE SPECIFICITY**
MUTIERTE CELLOBIOSE-DEHYDROGENASE MIT MODIFIZIERTER SUBSTRATSPEZIFITÄT
DÉSHYDROGÉNASE DE CELLOBIOSE MUTÉE DOTÉE D'UNE SPÉCIFICITÉ DE SUBSTRAT MODIFIÉE

(30) Priority: 25.07.2017 EP 17182992
(43) Date of publication of application: 03.06.2020
(73) Proprietor: DirectSens GmbH, 3400 Klosterneuburg (AT)
(72) Inventor: KITTL, Roman, 3400 Klosterneuburg (AT); BRESLMAYR, Erik, 3400 Klosterneuburg (AT); FELICE, Alfons, 3400 Klosterneuburg (AT)
(74) Representative: Loidl, Manuela Bettina
(86) International application number: PCT/EP2018/070131
(87) International publication number: WO 2019/020673

(56) References cited:
- EP-A1- 2 223 936
- EP-A1- 2 636 733
- WO-A1-2015/109405
- WO-A1-2016/090472
- WO-A1-2016/090473
- WO-A1-2017/129637
- DATABASE UniProt [Online] 12 April 2017 (2017-04-12), Gan P.: "Cellobiose dehydrogenase, Colletotrichum incanum", XP002784727, Database accession no. A0A1S1VN04
- DESRIANI ET AL: "Amino acid substitution at the substrate-binding subsite alters the specificity of the Phanerochaete chrysosporium cellobiose dehydrogenase", BIOCHEMICAL AND BIOPHYSICAL RESEARCH COMMUNICATIONS, ELSEVIER, AMSTERDAM NL, vol. 391, no. 2, 8 January 2010 (2010-01-08), pages 1246-1250, XP026831958, ISSN: 0006-291X, DOI: 10.1016/J.BBRC.2009.12.052 [retrieved on 2009-12-17]

## Description

The field of the present invention relates to recombinant enzyme modification to modify substrate specificity.

Cellobiose dehydrogenase (EC 1.1.99.18, CDH) was first discovered in 1974 in the extracellular enzyme system of *Phanerochaete chrysosporium* and later on in several other basidiomycetous fungi. A special characteristic of this enzyme is its composition: the combination of a catalytically active flavodehydrogenase domain (also called "flavin domain"), hosting a non-covalently bound FAD, and a haem domain, with a haem b as a cofactor. Both domains are connected by a linker. By its catalytic activity the natural substrate cellobiose is oxidised in a reaction which reduces the FAD of the flavin domain.

CDH or its flavodehydrogenase domain oxidises carbohydrates like its natural substrates cellobiose and cello-oligosaccharides and others like lactose and maltose. CDHs have been discovered and shown previously to be capable of converting glucose efficiently (Harreither et al., 2011; WO 2010/097462 A). Modified CDHs exist that have reduced activity on maltose (WO2013/131942). Other CDHS are known from *Metarhizium anisopliae* (Database Uniprot, acc. No. E9DZA5) or *Stemphylium lycopersici* (Database Uniprot, acc. No. A0A0L1HDV7). Gao et al. (Plos Genetics, 7 (1), (2001): e1001264) describe genomic sequencing of the fungi *Metarhizium anisopliae* and *M*. *acridum.* EP 2 636 733 A1 describes artificially mutated CDHs to reduce its maltose oxidation activity. Desriani et al. (Biochemical and Biophysical Research Communications, 391 (2010), 1246-1250) discloses mutations of the active site of the CDH of Phanerochaete chrysosporium and studied the effect of Glu279 substitutions on enzyme activity and substrate specificity.

The problem with CDHs lies in the versatility which diminishes its use in sensors for detecting single analyte in mixtures of several potential substrates.

It is a goal of the present invention to provide a cellobiose dehydrogenase or its catalytically active flavodehydrogenase domain, which is capable to selectively detect lactose in the presence of galacto-oligosaccharides especially with direct electron transfer based electrodes or mediated electron transfer-based electrodes to provide suitable analytically useful sensors.

The present invention relates to recombinant modified cellobiose dehydrogenases (CDH) with reduced galacto-oligosaccharide turnover, especially of 6-galactosyllactose or 4-galactosyllactose turnover. The aim is to reduce the effect of any galacto-oligosaccharide concentration, but preferably at least of 6-galactosyllactose or 4-galactosyllactose or both, present in a sample matrix on lactose detection. In particular, described is a modified cellobiose dehydrogenase (CDH) or its functional flavodehydrogenase domain having a substitu- tion at least at one of the amino acids of the galacto- oligosaccharide binding motif corresponding to amino acids 555- 557 and 639-641 of SEQ ID NO: 4 (CDH from *N. crassa)* or corresponding to amino acids 554-556 and 638-640 of SEQ ID NO: 5 (CDH from M. *thermophilum)* by any other acid amino ac- id.

The amino acids corresponding to amino acids 555-557, 627-629 and 639-641 of SEQ ID NO: 4 are in case of SEQ ID NO :4 and other ascomycota N555-D557 (amino acid sequence NTD, also referred to as "NTD-part" or "GOS motif l"), S627-E629 (amino acid sequence SFE, also referred to as "SFE-part" or "GOS motif 3") and S639-Y641 (amino acid sequence SQY, also referred to as "SQY-part" or "GOS motif 2") of SEQ ID NO: 4. These amino acids may vary in different CDHs, especially in basidiomycota. E.g., the NTD-part of the motif in SEQ ID NO: 1 could be changed to NTE, NTV or NTE and/or the SQY-part of the motif in SEQ ID NO: 4 could be changed to NQY. In the course of the present invention, this GOS motif was identified as being responsible for galacto-oligiosaccharide binding for oxidation by the CDH independent of smaller substrates like lactose. The GOS motif comprises three parts which form a three-dimensional structure with the three parts in vicinity to each other.

The invention may further comprise an amino acid substitution at the amino acid corresponding to N721 of SEQ ID NO: 5 (CDH from M. *thermophilum)* or corresponding to N722 of SEQ ID NO: 4 (CDH from *N. crassa)* by glutamine, isoleucine, threonine or leucine or a substitution of the asparagine in the active center motif having the amino acid sequence NHW by glutamine, isoleucine, threonine or leucine; hence this motif would be QHW, IHW, THW or LHW, as e.g. described in PCT/EP2017/051577 (WO 2017/129637). Usually, the NHW-motif is found near the C-terminus of a CDH or the flavodehydrogenase domain, e.g. around amino acids 660 to 780 of a CDH or amino acids 430 to 550 of a flavodehydrogenase domain. Such a mutation reduces glucose or galactose oxidation activity as described in PCT/EP2017/051577 (WO 2017/129637). Glucose and galactose may be sources of further unwanted cross-reaction in lactose detection and said cross-reactivity may be reduced with such a substitution corresponding to N721 of SEQ ID NO: 5 or at the NHW motif.

Preferred modified cellobiose dehydrogenase enzymes of the invention are optimized for use in biosensors based on direct electron transfer (3^{rd} generation) or based on mediated electron transfer (2^{nd} generation) .

To increase the performance of CDH as selective electrode catalyst for lactose, the reduction of galacto-oligosaccharide oxidation activity alone or in combination with an increase of lactose oxidation activity was pursued by genetic modification. With the general description of modifying a CDHs flavodehydrogenase domain, a domain of high homology in all CDHs, it is possible to modify any CDH according to the principles outlined herein in order to decrease galacto-oligosaccharide sensitivity. Thus, the present invention provides in particular a modified cellobiose dehydrogenase (CDH) or its functional flavodehydrogenase domain having reduced galacto-oligosaccharide oxidation activity as compared to the unmodified CDH or its functional flavodehydrogenase domain while essentially maintaining a high lactose oxidation activity, e.g. at most a 40% or at most a 20% reduction in lactose oxidation activity, at standard conditions and as shown in the examples. The inventive substitutions may reduce the activity of lactose oxidation as long as the ratio of lactose oxidation to an oxidation of a galacto-oligosaccharide is increased. This increase in ratio or specificity achieves the goal of reduced side-reactivities in the detection of lactose. The invention further provides a method of oxidizing lactose with the CDH of the invention. Such a method is preferably used in the analytical detection of lactose in a sample. Also possible is the detection of cellobiose or maltose.

Most CDHs are capable of oxidizing lactose. The oxidation reaction can be detected e.g. by monitoring electron acceptors for the redox reaction such quinones, like as DCIP (2,6-dichloroindophenol) *o*- or *p*-benzoquinone or derivatives thereof, methylene blue, methylene green, Meldola's blue, potassium ferricyanide, ferricenium hexafluorophosphate, FeCl₃ or cytochrome c (the latter being a haem domain cofactor) or simply by determining electric current or voltages on an electrode, the electron acceptor being the electrode surface.

It is not necessary to use an entire CDH; the flavin domain, even without the haem domain, is sufficient for catalytical activity. The domain is therefore referred to as "functional domain" as it has the function of oxidizing lactose with a suitable electron acceptor. The activity is exerted by either the whole enzyme cellobiose dehydrogenase or the catalytically active flavodehydrogenase domain.

Wild type CDHs have an undesirable activity to oxidize galacto-oligosaccharides especially when they are structurally similar to lactose (e.g.: 6-galactosyllactose, 4-glactosyllactose). The modification according to the present invention should now be understood in that the inventive CDHs deviate from the wild-type CDHs by this substantially decreased galacto-oligosaccharide oxidation activity. This substantially decreased galacto-oligosaccharide oxidation activity may be in combination with an increased or even a decrease in lactose oxidation activity, as mentioned above. The invention increases the ratio of the lactose oxidation activity to the galacto-oligosaccharide oxidation activity. The ratio of lactose oxidation activity to the oxidation activity of 6-galactosyllactose is at least 2:1, preferably at least 3:1, at comparable conditions, as e.g. described in the examples, in particular at 23°C and atmospheric pressure in aqueous solution.

Preferred modified CDHs or their functional flavodehydrogenase domain are of a CDH of the subkingdom *dikarya.* Preferably, the CDH is from Ascomycota or Basidiomycota. Preferred ascomycota are selected from *Myriococcum thermophilum*, *Corynascus thermophilus, Chaetomium atrobrunneum (Myceliophthora fergusii), Hypoxylon haematostroma, Neurospora crassa, Stachybotrys bisby* or *Humicola insolens.* Preferred Basidiomycota are *Athelia rolfsii, Gelatoporia subvermispora, Phanerochaete chrysosporium, Trametes versicolor.* Ascomycota are particularly preferred according to the invention. Such unmodified CDHs are described in WO 2010/097462 A and in WO2013/131942, including encoding nucleotide sequences, and in sequences of SEQ ID NO: 1-10 herein. Suitable sequences are also available in sequence databases, in particular the NCBI database, e.g. accession numbers ADT70773.1, ADT70775.1, ADT70772.1, XP_956591.1, ABS45567.2, ADT70777.1, AAO64483.1, ACF60617.1, AAB61455.1, XP_008041466.1, AAF69005. "Unmodified" as used herein is a CDH without the inventive modification that decreases galacto-oligosaccharide oxidation activity. There may be further modifications that increase interaction between the flavin and the haem domain if an entire CDH is used (such as described in WO 2010/097462) or a modification that reduce glucose oxidation activity in relation with lactose oxidation activity as described in PCT/EP2017/051577 (WO 2017/129637) .

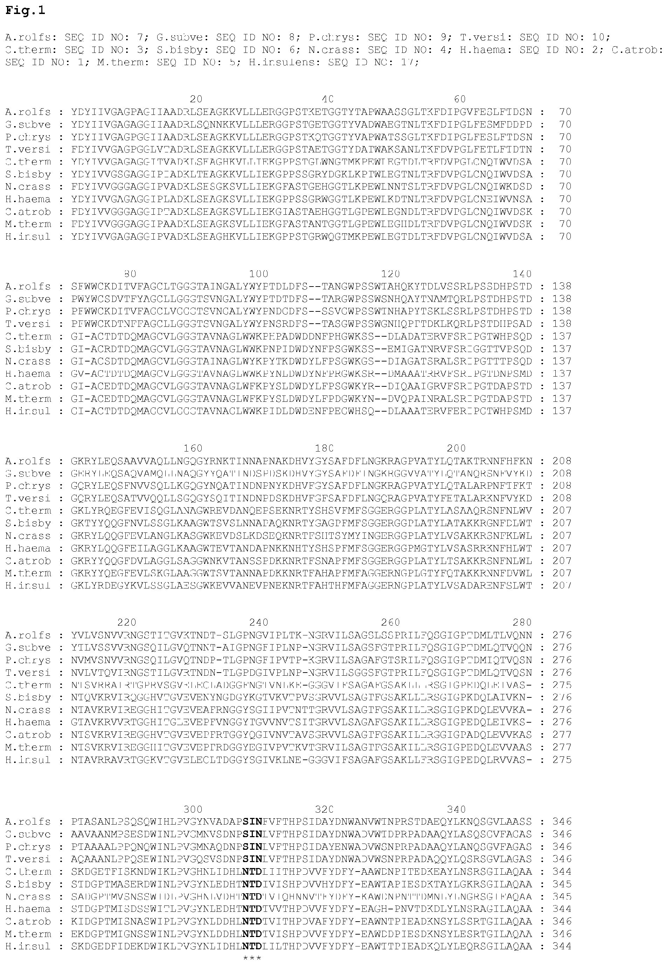

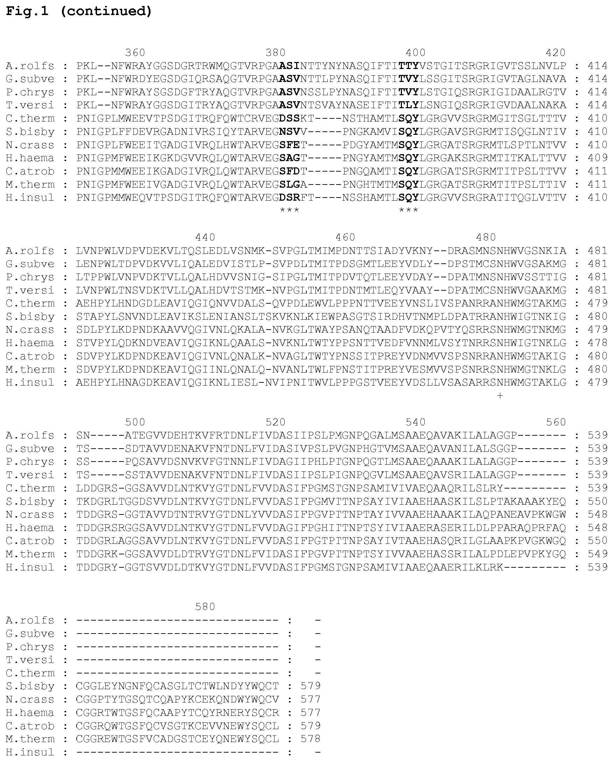

The inventive CDH or flavodehydrogenase domain has a substitution at least at one of the amino acids of the galacto-oligosaccharide binding motif corresponding to amino acids 555, 556, 557, 639, 640 and 641 of SEQ ID NO: 4 (CDH from N. crassa). Regarding other CDH sequences provided herein, the galacto-oligosaccharide binding motif (GOS motif) corresponding to amino acids 555-557 and 639-641 of SEQ ID NO: 4 may be found at amino acids 556-558 and 640-642 of SEQ ID NO: 1, amino acids 571-573 and 655-657 of SEQ ID NO: 2, amino acids 550-552 and 635-637 of SEQ ID NO: 3, amino acids 554-556 and 638-640 of SEQ ID NO: 5, amino acids 554-556 and 638-640 of SEQ ID NO: 6, amino acids 548-550,
and 633-635 of SEQ ID NO: 17 (see alignments at Fig. 1-4, note Fig. 1 only shows the flavodehydrogenase domain and is numbered accordingly).

A preferred selection of amino acids to be substituted are those at an amino acid corresponding to N555, D557 and S639 of the CDH of SEQ ID NO: 4. Substitutions at these sites are particularly effective.

The invention has shown that any substitution at these positions, introducing a change to the wild type sequence as e.g. found in SEQ ID NO: 1-6, reduces galacto-oligosaccharide oxidation activity, as such or in relation to lactose. Therefore, any substitution is possible to achieve the inventive goal of a robust lactose oxidation with less interference from galacto-oligosaccharides. Some substitutions are of course more effective than others. Preferred substations are by at least one amino acid change corresponding to a substitution selected from D557V, D557L, S639V, S639N, N555M, S639I, D557R, S639G, D557T, D557I, D557E, N555L, D557P, S639M, S639R, D557G, S639E, D557A, S639Q, S639K, S639C, D557S, S639D, D557M, D557H, S639W, D557W, S639F, N555D, D557Q, S639Y, S639L, N555K, S639H, D557C of the CDH of SEQ ID NO: 4.

In further preferred embodiments of the invention, an amino acid corresponding to amino acid 557 of SEQID NO: 4 is preferably substituted by any non-aspartic acid natural amino acid, such as by glutamic acid, valine, or asparagine. An amino acid corresponding to amino acid 639 of SEQ ID NO: 4 (CDH from N. *crassa*) or corresponding amino acid 638 of SEQ ID NO: 5 (CDH from M. *thermophilum)* is preferably substituted by any natural non-serine amino acid, such as by glutamic acid, valine, or asparagine. Of course, such changes apply to all CDHs, preferably those related to SEQ ID NO: 1-6 and 17.

As said a preferred further substitution increases the ratio of lactose oxidation to glucose or galactose oxidation. Preferably the modified CDH or its functional flavodehydrogenase domain is based on an unmodified CDH or flavodehydrogenase domain with an active center motif having the sequence X₁X₂NHWX₃X₄X₅, wherein X₁ is any amino acid, preferably selected from N, C and R; X₂ is selected from S and A; X₃ is selected from V, M and I; X₄ is any amino acid, preferably selected from G and S; and X₅ is any amino acid, preferably selected from S, A and T; especially preferred X₄ and X₅ are not both S. According to this additional substitution, the N in this motif is preferably changed to Q, I, T or L. This change facilitates the activity change reducing activity on glucose and/or galactose. With the information of the motif, the position for the substitution can be determined. Preferably, also the modified CDH or the domain comprises the sequence X₁X₂ZHWX₃X₄X₅, with Z, being Q, I, T or L and X₁-X₅ being defined as above. Preferably, the amino acid corresponding to N721 of SEQ ID NO: 5 or asparagine at the NHW motif is amino acid N723 of SEQ ID NO: 1, N738 of SEQ ID NO: 2, N718 of SEQ ID NO: 3, N722 of SEQ ID NO: 4, N721 of SEQ ID NO: 5, N721 of SEQ ID NO: 6, N716 of SEQ ID NO: 17.

The modified CDH or its functional flavodehydrogenase domain preferably comprises a modified flavodehydrogenase domain based on one of the unmodified flavodehydrogenase domains according to amino acids 253-831 of SEQ ID NO: 1, amino acids 269-845 of SEQ ID NO: 2, amino acids 249-787 of SEQ ID NO: 3, amino acids 253-829 of SEQ ID NO: 4, amino acids 251-828 of SEQ ID NO: 5, amino acids 251-829 of SEQ ID NO: 6, amino acids 246-785 of SEQ ID NO: 17. Preferably the inventive modified fla- vodehydrogenase domain has a sequence with at least 75%, at least 80%, at least 85%, at least 90%, at least 95%, at least 98%, in particular preferred at least 99%, se- quence identity with one of said unmodified flavodehydrogenase domains and further comprises at least one amino acid substitu- tion at the amino acid corresponding to amino acids 555-557 and 639-641 of SEQ ID NO: 4 or at the GOS motif, suitable for reducing the galacto-oligosaccharide oxidation activity.

Homologous CDHs or flavodehydrogenase domains within these sequence requirements can be readily identified by sequence comparisons such as by sequence alignment using publicly available tools, such as BLASTP, ClustalO, ClustalW or FastDB. Preferably, a homologous or modified CDH or the domain thereof has 1, 2, 3, 4, 5, 6, 7, 8, 9, 10, at least 11, at last 13, at least 15, at least 17, at least 20, at least 25, at least 30, at least 40, at least 50, at least 60, at least 80, at least 100 and/or up to 100, up to 80, up to 60, up to 50, up to 40, up to 30, up to 30, up to 20, up to 15 further amino acid substitutions, deletions, insertions or modifications, and any ranges between these values, as compared to any one of the CDHs of SEQ ID NOs 1-6 or any of their flavodehydrogenase domains. A particular preferred base CDH is of *N. crassa,* SEQ ID NO: 4. A prediction of such modifications can be made by computational methods using e.g. molecular docking into crystal structures such as of PDB database entry "lkdg" or "4qi5". Preferably, the inventive CDH or flavodehydrogenase domain has 1 substitution at the GOS motif, preferably at its parts GOS motif 1 or GOS motif 2; but further mutations in this motif and its -1 or -2 parts are possible, such as 2, 3, 4, 5, or more substitutions. Such combinations are e.g. one substitution in each of the GOS motif-1 and GOS motif-2 parts or one substitution in each of the three parts.

Further modifications outside the GOS motif are possible. The effect of such further modified amino acids in the active site and the substrate binding site can be determined for homogeneous catalysis by photometric methods and for heterogeneous catalysis by electrochemical measurements using enzyme electrodes as described herein.

The methods for further modification may be any known in the art such as amino acid mutations, including amino acid substitutions, deletions or additions but also chemical modification/derivatisation of amino acid side chains.

The inventive enzyme or domain is usually recombinantly expressed. Also provided are preparations comprising the modified CDH or domain. The term "enzyme" or "enzyme preparation" as used herein refers to a cellobiose dehydrogenase or its flavodehydrogenase domain from a specified organism which is at least about 20% pure, preferably at least about 40% pure, even more preferably at least about 60% pure, even more preferably at least 80% pure and most preferably at least 90% pure, as determined by polyacrylamide gel electrophoresis (SDS-PAGE).

The present invention relates to modified/genetically engineered cellobiose dehydrogenases or flavodehydrogenase domain from existing protein scaffolds, which oxidise galacto-oligosaccharides less efficiently with or without a more efficient lactose turnover than the currently known cellobiose dehydrogenases, especially those that is closest related to the modified CDH or the flavodehydrogenase domain, such as a CDH or flavodehydrogenase domain of sequences SEQ ID NO: 1-10 and 17 (the portions for the flavodehydrogenase domain are given below in the description of Fig. 1).

Of course, the modified CDH or the flavin domain (aka flavodehydrogenase domain) may still have activity for an electro-catalytic oxidation of galacto-oligosaccharides. It is sufficient that the concentration dependence of the signal from galacto-oligosaccharides is reduced. Essentially constant background signals dependent on galacto-oligosaccharides can be removed from the lactose detection by normalization. Especially preferred the CDH or flavin domain has the property that a signal of galacto-oligosaccharides during lactose detection or lactose concentration determination is below 40%, preferably below 20%; particularly the signal, e.g. electrode current or electrochemical reduction of an electron acceptor, of 0.25 g/L 6-galactosyllactose or 4-galactosyllactose in a solution compared to the signal of a 0.25 g/L lactose solution is below 40%, preferably below 20%. In preferred embodiments of the invention the 6-galactosyllactose or 4-galactosyllactose oxidation activity of the CDH or the domain is reduced in relation to lactose oxidation activity. Especially preferred the concentration dependency of the 6-galactosyllactose or 4-galactosyllactose oxidation is reduced so that - if 6-galactosyllactose or 4-galactosyllactose is present - only a substantially constant contribution of 6-galactosyllactose or 4-galactosyllactose to the signal is detected in relation to a lactose signal.

It is understood that one of skill in the art may engineer the mentioned or other cellobiose dehydrogenases to obtain the modified CDH or the active flavodehydrogenase domain using the principles outlined herein or in the prior art (WO 2010/097462 A, WO2013/131942) like the rational enzyme engineering via site-directed mutagenesis or directed evolution approaches (e.g., gene shuffling, error-prone PCR, etc.) and subsequent screening of the generated diversity. The techniques to introduce a further mutation into the nucleic acid sequence to exchange one nucleotide for another nucleotide with the aim to exchange one amino acid for another in the resulting protein may be accomplished by site-directed mutagenesis using any of the methods known in the art. The amino acid positions and modification to modify a CDH can be obtained by homology modelling using the crystal structure of *Phanerochaete chrysosporium* CDH (PDB database entry 1kdg) as template and superimposition of the obtained models as well as docking studies. It is possible to use a CDH as template and by sequence comparison to obtain corresponding amino acids for modification to reduce galacto-oligosaccharide oxidation activity.

The modified CDH or its functional flavodehydrogenase domain may be isolated by diafiltration, ion exchange chromatography and preferably being further purified by hydrophobic interaction chromatography. Preferably the CDH or the domain is recombinantly produced by *Pichia pastoris.*

The invention further provides a nucleic acid molecule encoding a modified CDH or its functional flavodehydrogenase domain as described above. Unmodified CDH sequences are known in the art, e.g. in the NCBI sequence database and disclosed in WO 2010/097462 A and WO2013/131942. The nucleic acid can be modified to encode the inventive CDH or flavin domain with the substitution or further mutation. The nucleic acids, CDH or domains described herein may be iso- lated and/or purified.

Further provided is a method of producing a modified CDH or its functional flavodehydrogenase domain of the invention, comprising recombinantly expressing a nucleic acid molecule encoding said modified CDH or its functional flavodehydrogenase domain in a host cell.

In a further aspect, the invention further provides an electrode comprising an immobilised cellobiose dehydrogenase or its functional flavodehydrogenase domain of the invention.

Preferably the electrode comprises an immobilised CDH in direct- or mediated electron transfer mode (Tasca et al. 2011a, Tasca et al. 2011b, Ludwig et al. 2010, Safina et al. 2010, Tasca et al 2010a, Tasca et al. 2010b) or an immobilised flavodehydrogenase domain in mediated electron transfer mode. As electrode, any suitable surface for collecting electrons from CDH is understood. The electrode may be of any material suitable to immobilise the CDH, e.g. carbon such as graphite, pyrolytic graphite, glassy carbon, carbon nanotubes (single or multi-walled), carbon fibres, boron doped diamond, gold electrodes modified with promoters e.g., thiols or screen-printed electrodes. This is a non-exhaustive list of possible electrodes, which may e.g. contain other nanoparticles (gold,...) to increase the specific surface area. Particular uses of the inventive electrodes are in the provision of biosensors, more specifically to lactose biosensors using the direct electron transfer properties (DET) of cellobiose dehydrogenase (CDH) or using mediated electron transfer properties (MET) to measure the lactose concentration at acidic, neutral, alkaline pH.

On the electrode, the CDH or the flavodehydrogenase domain may be immobilised by adsorption, preferably also physical entrapment in a polymer, complex formation, preferably via an additional complexing linker, covalent binding, in particular cross-linking, or ionic binding and/or the immobilized cellobiose dehydrogenase can be cross-linked, in particular by bifunctional agents, to increase stability or activity. It has been shown that cross-linking with bifunctional agents, such as agents with two reactive groups making a connection with the CDH, can stabilize the CDH and even increase its activity on graphite electrodes measurable by amperometric methods described herein. This advantage can lead to an increased sensitivity and lowering the detection limit for lactose. Such a cross-linking agent is e.g. glutaraldehyde or any other dialdehydes. Further methods for immobilization are described in e.g. WO 2010/097462 and WO2013/131942, which can be used according to the invention.

The electrodes might be used in form of a single electrode or electrode stacks, e.g. of 2, 3, 4, or more electrodes.

Electrode configurations and uses, including measurement parameters are described in e.g. WO 2010/097462 and WO2013/131942, which can be used according to the invention.

The invention further provides a method of oxidizing lactose with the inventive CDH or flavin domain, especially in a method of detecting or quantifying lactose in a sample comprising the step of oxidizing lactose in said sample with a modified CDH or its functional flavodehydrogenase domain or an electrode as described herein and detecting or quantifying said oxidation, preferably wherein said sample comprises or is suspected of comprising a galacto-oligosaccharide, such as 6-galactosyllactose or 4-galactosyllactose. The fluid sample may be any fluid which potentially comprises lactose, including milk or milk or dairy products, such as whey or cheese. If solid products are analysed, such as cheese, the carbohydrates, including lactose, may be extracted or the solid product may be fluidized, such as by dissolving.

In a further aspect, the present invention provides a lactose assay kit comprising the modified cellobiose dehydrogenase or its functional flavodehydrogenase domain or an electrode as described herein. The kit may in preferred embodiments also comprise auxiliary substances, like buffers, and containers such as a sample holding means and/or lactose standards. Lactose standards may be used to calibrate the assay. The kit may also comprise a reader for a signal, especially an electrochemical signal such as a potentiostat, a computer readable memory device with software for calibration and/or measurement calculations.

According to the invention there is provided a modified cellobiose dehydrogenase (CDH) or its functional flavodehydrogenase domain having a substitution at least at one of the amino acids of the galacto-oligosaccharide binding motif corresponding to amino acids 555-557 and 639-641 of SEQ ID NO: 4 (CDH from *N. crassa)* and comprising a modified flavodehydrogenase domain based on one of the unmodified flavodehydrogenase domains according to amino acids 253-831 of SEQ ID NO: 1, amino acids 269-845 of SEQ ID NO: 2, amino acids 249-787 of SEQ ID NO: 3, amino acids 253-829 of SEQ ID NO: 4, amino acids 251-828 of SEQ ID NO: 5, amino acids 251-829 of SEQ ID NO: 6, amino acids 246-785 of SEQ ID NO: 17; said modified flavodehydrogenase domain has a sequence with at least 75% sequence identity with one of said unmodified flavodehydrogenase domains, wherein the ratio of lactose oxidation activity to the oxidation activity of 6- galactosyllactose is at least 2:1.

According to the invention there is further provided a nucleic acid molecule encoding a modified CDH or its functional flavodehydrogenase domain.

According to the invention there is further provided a method of producing a modified CDH or its functional flavodehydrogenase domain described herein, comprising recombinantly expressing a nucleic acid molecule in a host cell.

According to the invention there is further provided an electrode comprising an immobilised cellobiose dehy- drogenase or its functional flavodehydrogenase domain; preferably wherein the cellobiose dehydrogenase is immobilised by adsorption, complex formation, especially preferred via an additional complexing linker, covalent or ionic linkage, and/or wherein preferably the immobilized cellobiose dehydrogenase is cross-linked, in particular by bifunctional agents, to increase stability or activity.

According to the invention there is further provided a method of detecting or quantifying lactose in a sample comprising the step of oxidizing lactose in said sample with a modified CDH or its functional flavodehydrogenase domain, or an electrode and detecting or quantifying said oxidation, preferably wherein said sample comprises or is suspected of comprising a galacto- oligosaccharide, especially preferred a milk or milk product containing sample.

According to the invention there is further provided a lactose assay kit comprising the modified Cellobiose dehydrogenase or its functional flavodehydrogenase domain or an electrode and a sample holding means and/or lactose standards.

The present invention is further illustrated by the following figures and examples without being restricted thereto.

### Figures:

**Figure 1** is a sequence alignment of amino acid sequences of the flavodehydrogenase domains ("flavin domains") of the wild-type CDHs from *Chaetomium atrobrunneum* (aa 253-831 of SEQ ID NO: 1), *Hypoxylon haematostroma* (aa 269-845 of SEQ ID NO: 2), *Corynascus thermophilus* (aa 249-787 of SEQ ID NO: 3), *Neurospora crassa* (aa 253-829 of SEQ ID NO: 4), *Myriococcum thermophilum* (aa 251-828 of SEQ ID NO: 5), *Stachybotrys bisby* (aa 251-829 of SEQ ID NO: 6), *Athelia rolfsii* (aa 233-771 of SEQ ID NO: 7), *Gelatoporia subvermispora* (aa 236-774 of SEQ ID NO: 8), *Phanerochaete chrysosporium* (aa 235-773 of SEQ ID NO: 9), *Trametes versicolor* (aa 230-768 of SEQ ID NO: 10), *Humicola insolens* (aa 246-785 of SEQ ID NO: 17). The mutation site at the galacto-oligosaccharide (GOS) binding motif corresponding to amino acids 555-557, 627-629 and 639-641 of SEQ ID NO: 4 are marked by "*". An optional additional mutation site relating to glucose/lactose sensitivity at the amino acid corresponding to N72l of SEQ ID NO: 5 is highlighted and marked by "+" (position 483 of the shown consensus numbering of the shown flavin domains).

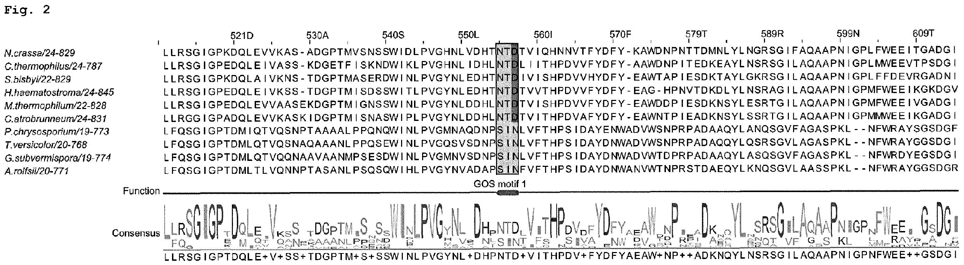

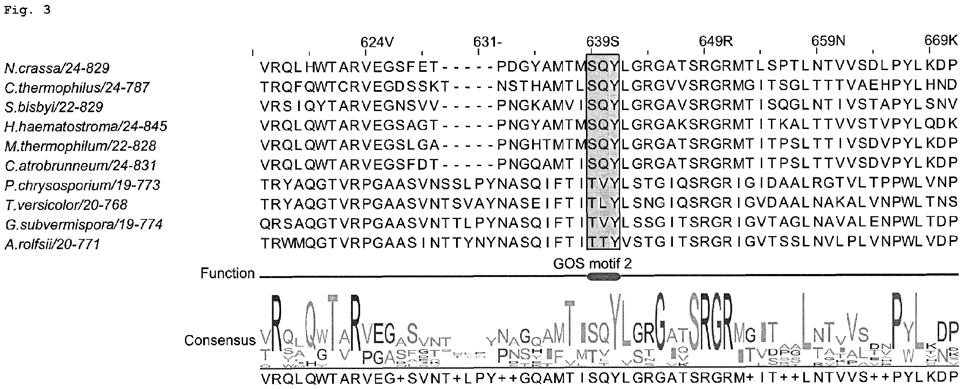

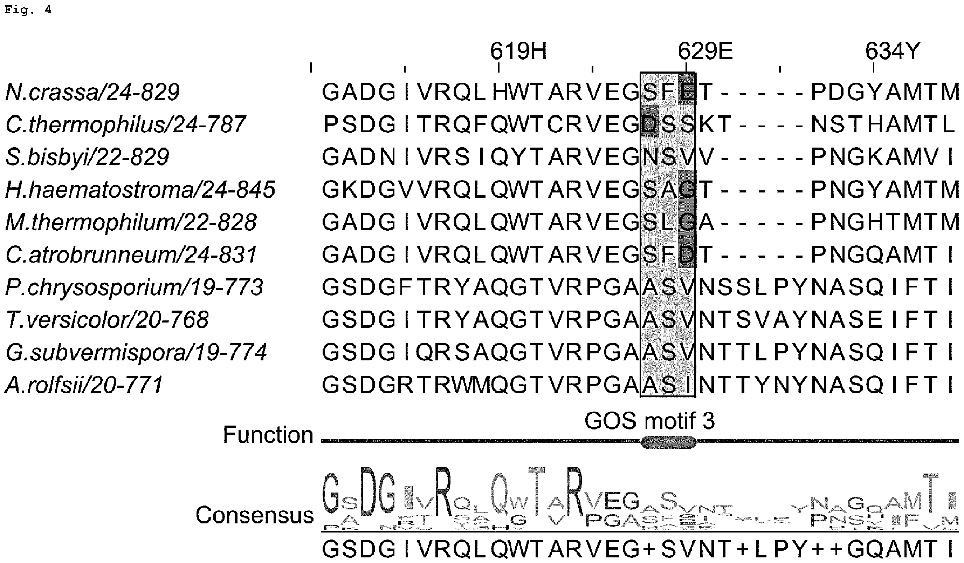

**Figure 2** shows a sequence alignment of the CDHs at the position of the GOS motif 1. **Figure 3** shows a sequence alignment of the CDHs at the position of the GOS motif 2. **Figure 4** shows a sequence alignment of the CDHs at the position of the GOS motif 3. Numbering corresponding SEQ ID NO:4 was used.

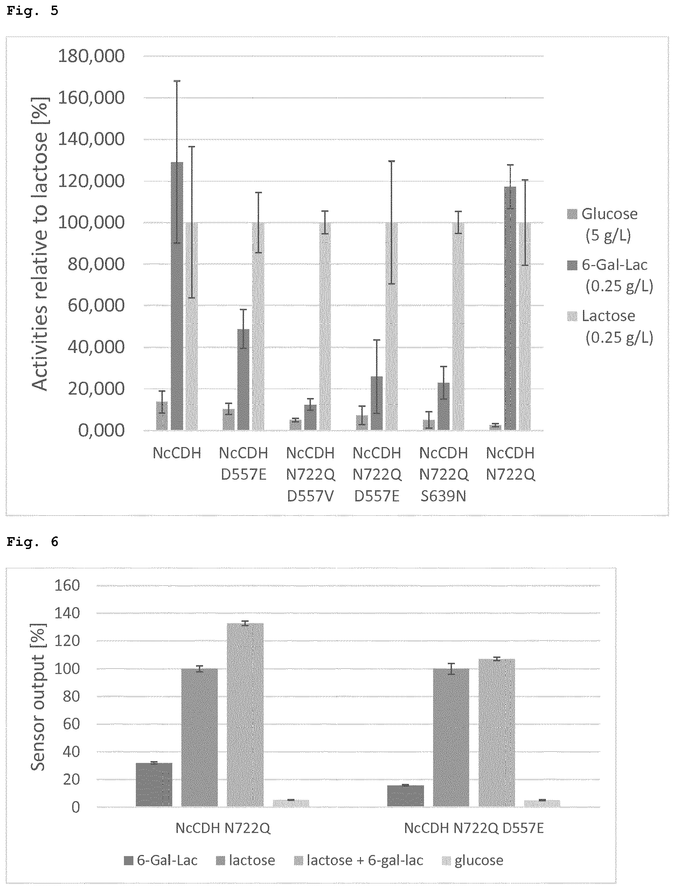

**Figure 5** shows activities of the supernatants of the cultures NcCDH, NcCDH D557V, NcCDH N722Q D557V, NcCDH N722Q D557E, NcCDH N722Q S639N, and NcCDH N722Q measured with the cyt c assay with 5 g L⁻¹ glucose, 0.25 g^{-L} lactose or 0.25 g^{-L} 6-gal-lac. The activities are normalized to the average activity with lactose of each enzyme variant.
**Figure 6** shows the sensor output of the enzyme variant NcCDH N722Q D557E and its parent NcCDH N722Q with solutions of 0.25 g L⁻¹ lactose, 0.25 g L⁻¹ 6-gal-lac, 5 g L-1 glucose and of a combination of 0.25 g L⁻¹ lactose and 0.25 g L⁻¹ 6-gal-lac. The activities are normalized to the average activity with lactose of each enzyme variant.

### Examples:

### Example 1: Materials

Chemicals used in buffers and fermentation media were commercial products and at least of analytical grade if not otherwise stated. Substrates for kinetic studies were lactose, glucose and cytochrome c (cyt c) from Sigma-Aldrich and 6'-galactosyllactose (6-gal-lac) from Carbosynth in the highest grade of purity available. Buffers were prepared using water purified and deionised (18 MΩ) with a Milli-Q system (Millipore, Bedford, MA, USA) .

### Example 2: Enzymatic activity assays and steady-state kinetics

Enzyme activity was assayed at 30°C using cyt c (Canevascini et al.,1991) as electron acceptor. Stock solutions of carbohydrates used for kinetic measurements were prepared in the respective buffer and allowed to stand overnight for mutarotation, while stock solutions of electron acceptors were prepared in water and immediately used. The protein concentration was determined with the Bradford assay.

### Example 3: Protein characterisation

The protein concentration was determined by the dye-staining method of Bradford using a pre-fabricated assay from Bio-Rad Laboratories Hercules, CA, USA) and bovine serum albumin as standard according to the manufacturers recommendations.

For electrophoretic characterisation SDS-PAGE was carried out on a Hoefer SE 260 Mighty Small II vertical electrophoresis unit. Gels (10.5x10 cm; 10% T, 2.7% C) were cast and run according to the manufacturers' modifications of the Laemmli system.

### Example 4: Docking study

A 6-galactosyl-lactose tri-saccharide structure was docked to the structure of CDH form *Myriococcum thermophilium* (PDB database entry 4qi5). Docking studies were performed using Molecular Operating Environment (MOE) software (Chemical Computing Group, Quebec, Canada). Quality was assessed by comparison of the docking position of the 6-galactosly-lactose molecule to the cellobionolactam molecule co-crystalized in the 4qi5 structure. The best fit was chosen for further investigation. Three regions (GOS motif 1 (*N.crassa*_N555-D557), GOS motif 2 (*N.crassa*_S639-Y641), GOS motif 3 (*N.crassa*_S627-E629)) defined by amino acid residues in close vicinity to the modeled galactosyl residue were selected and in silico site saturation mutagenesis was performed. Best results judged by theoretical binding affinity and stability values calculated by the software were selected for expression studies. Amino acids N555, D557, S627 and S639 were preliminarily closer investigated. In descending order, the following amino acid substitutions reduce GOS binding to the CDH: D557V, D557L, S627D, S639V, S639N, N555M, S639I, D557R, S639G, D557T, D557I, D557E, S627K, S627T, S627P, N555L, D557P, S639M, S639R, D557G, S639E, D557A, S639Q, S639K, S639C, D557S, S639D, D557M, D557H, S639W, S627Q, D557W, S639F, N555D, S627Y, D557Q, S639Y, S639L, N555K, S639H, S627W, D557C (all amino acid designations with regard to SEQ ID NO: 4, the *N. crassa).* Surprisingly, various substitute amino acids with various properties (acidic, basic, aliphatic, aromatic, hydroxylic, sulphurous, amidic, large, small) decrease affinity. This means that the GOS motif is in wild type CDH optimized for GOS binding and any change can reduce or disrupt such binding. Promising candidates were selected for wet-chemical validation.

### Example 5: Generation of Neurospora crassa CDH variants by site-directed mutagenesis

The amino acid exchanges D557E, D557V and S639N were selected for site-directed mutagenesis. The previously reported plasmid pNCIIA, encoding the *N. crassa* CDH gene (XM_951498, SEQ ID NO: 12 of WO 2010/097462) with its native signal sequences cloned under the control of the metha- nol-inducible AOX1 promoter was used as templates for the ampli- fication of the *target gene* with primers PR11 (5'- CAACACCGAAACCGTCATCCAGC-3', SEQ ID NO: ll) and PR12 (5'- GATGACGGTTTCGGTGTTGGTG-3', SEQ ID NO: l2) for mutation D557E. The plasmid pDS63, encoding the *N. crassa* CDH gene (XM_951498, SEQ ID NO: 12 of WO 2010/097462) with its native signal sequences cloned under the control of the methanol-inducible AOX1 promoter, modified with the amino acid exchange N722Q was used as template for the amplification of the *target gene* with primers PR11 (5'- CAACACCGAAACCGTCATCCAGC-3', SEQ ID NO: l1) and PR12 (5'- GATGACGGTTTCGGTGTTGGTG-3', SEQ ID NO: l2) for mutation D557E; PR9 (5'-CAACACCGTTACCGTCATCCAGC-3', SEQ ID NO: l3)and PR10 (5'- GATGACGGTAACGGTGTTGGTG-3', SEQ ID NO: l4) for mutation D557V, and PR7 (5'-GCCATGACCATGAACCAGTACCTTGGCCGTGGC-3', SEQ ID NO: l5) and PR8 (5'-CATGGTCATGGCGTAGCCGTC-3', SEQ ID NO: l6) for muta-tion S639N. PCR was performed with Phusion high-fidelity DNA polymerase from New England BioLabs, a deoxynucleoside triphosphate (dNTP) mix from Fermentas, oligonucleotide primers from VBC Biotech (Vienna, Austria), and a C-1000 thermocycler from Bio-Rad Laboratories. The resulting PCR fragments were digested with *Dpn*I and transformed into *E.coli.* The correct sequences of the resulting plasmids were confirmed by sequencing. Linearized, verified plasmids were used for transformation into electrocompetent *P. pastoris* cells and transformants were selected on YPD Zeocin plates (100 mg L⁻¹).

### Example 6: Lactose to galacto-oligosaccharide activity

The resulting four *P. pastoris* clones expressing the enzyme variants NcCDH D557E, NcCDH N722Q D557E, NcCDH N722Q D557V and NcCDH N722Q S639N were produced together with the Wildtype NcCDH and the parent variant NcCDH N722Q in 96-well deep well plates according to methods described previously by Weis et al. (2004) . The activities of the supernatants of each cultivation were measured with the cyt c assay with 5 g L⁻¹ glucose, 0.25 g^{-L} lactose or 0.25 g^{-L} 6-gal-lac. 6-gal-lac was chosen as a representative of galacto-oligosaccharides (Sako et al., 1999), because of its strong presence in hydrolyzed low lactose milk, rendering it a significant substance for applications. Fig. 2 shows the results of the activity measurements and Table 1 shows the ratios of activities lactose:6-gal-lac and lactose:glucose.

**Table 1: Ratios of lactose to glucose or 6-gal-lac**

| | ratio lactose:glucose | ratio lactose:6-gal-lac |
|---|---|---|
| NcCDH | 7.3:1 | 0.8:1 |
| NcCDH D557E | 9.7:1 | 2.1:1 |
| NcCDH N722Q D557V | 20:1 | 8.1:1 |
| NcCDH N722Q D557E | 13.6:1 | 3.9:1 |
| NcCDH N722Q S639N | 19.7:1 | 4.4:1 |
| NcCDH N722Q | 40.3:1 | 0.9:1 |

The ratios lactose:6-gal-lac are strongly increased by the mutation; the ratios lactose:glucose are reduced, but still high enough for all variants with a substitution in the GOS motif.

### Example 7: Production of recombinant CDH

Parent enzyme NcCDH N722Q as reference and the variant NcCDH N722Q D557E were produced in 1L baffled flasks. Precultures were grown overnight in 30 mL of YPD medium at 30°C and 120 rpm. After approximately 18 hours the precultures were transferred into 1L baffled flasks containing 200 mL BMGY medium without methanol. Induction with methanol was started immediately using a multichannel peristaltic pump (Minipuls Evolution, Gilson, Middleton, WI, USA). Each flask was supplied eight times a day with methanol yielding a total concentration of 2% (v/v) methanol per day. Increase in activity was monitored using the DCIP and the cytochrome c enzyme assays. Cultivation was stopped at day five of methanol induction, cells removed by centrifugation (4000 rpm, 20 min) and the supernatant set to a final ammonium sulfate concentration of 20%.

### Example 8: Purification of recombinant CDH

Both enzymes were purified to homogeneity in a two-step purification. The sample was loaded on a 20 mL PHE Sepharose FF column (HR26/20) equilibrated with 50 mM Na-acetate buffer pH 5.5 containing 20% ammonium sulfate. Proteins were eluted by increasing the concentration of the elution buffer (50 mM Na-acetate buffer pH 5.5) from 0 to 100% in 5 column volumes and fractions containing CDH activity were pooled. After diafiltration with a polyethersulfone flat-stack cross flow module with a cut-off of 10 kDa (Viva Flow 50, Sartorius, Göttingen, Germany) until conductivity of 5 mS cm⁻¹ in 20 mM Na-acetate pH 5.5 the samples were loaded on a 20 mL Q-Source column (HR26/20) equilibrated with a 20 mM Na-acetate buffer pH 5.5. CDH was eluted by increasing the concentration of the elution buffer (50 mM Na-acetate buffer pH 5.5 containing 0.5 M NaCl) from 0 to 100% in 50 column volumes. Fractions were tested for CDH activity and pooled according to the highest Reinheitszahl (RZ, calculated from the absorbance ratio 420 nm/280 nm). Purified enzymes were concentrated and diafiltered in 50 mM citrate buffer, pH 5.5, aliquoted and kept at 4°C for further use.

### Example 9: Electrochemical Measurements

A screen-printed electrode with a three-electrode setup (Dropsens, Oviedo, Spain) was connected to an Autolab potentiostat (PGSTAT204, Metrohm Autolab B. V., Utrecht, The Netherlands) and used for electrochemical measurements. The enzyme-modified, screen-printed electrodes (DropSens, Oviedo, Spain) were mounted using an electrode holder (DropSens, Oviedo, Spain). 100µL drops of buffer containing various substrate concentrations were pipetted to cover all three electrodes. The measurements were started immediately. The system was controlled by the NOVA software version 1.11.0 (Metrohm Autolab B. V., Utrecht, The Netherlands).

### Example 10: Mutated CDHs from N. crassa - reduced galacto-oligosaccharide activity on electrodes

CDH from *N*. *crassa* oxidises besides lactose also glucose (Harreither et al., 2007) and galacto-oligosaccharides, which has negative side effects on the lactose detection accuracy if glucose and galacto-oligosaccharides are present. To reduce the activity with glucose, the mutation N722Q was previously introduced in *N*. *crassa* CDH. The enzyme variant NcCDH N722Q was nevertheless still active with galacto-oligosaccharides. Enzyme variants were designed to reduce this activity as described above. The molecular weights of variant NcCDH N722Q D557E did not differ significantly from the parent enzyme.

To investigate the electrochemical performance of the N. *crassa* CDH variant, the enzyme was immobilized on carbon electrodes and the current responses to different concentrations of glucose, 6-gal-lac and lactose were measured.
Figure 3 shows the current response to 0.25 g L⁻¹ lactose, 0.25 g L⁻¹ 6-gal-lac, 5 g L-1 glucose and of a combination of 0.25 g L⁻¹ lactose and 0.25 g L⁻¹ 6-gal-lac. Table 2 shows the ratios of activities lactose:6-gal-lac, lactose:glucose and lactose:lactose+6-gal-lac.

**Table 2: Ratios of lactose to glucose, 6-gal-lac, or lactose+6-gal-lac**

| | ratio lactose:glucose | ratio lactose:6-gal-lac | ratio lactose:lactose+6-gal-lac |
|---|---|---|---|
| NcCDH N722Q | 19.5:1 | 3.1:1 | 0.75:1 |
| NcCDH N722Q D557E | 19.7:1 | 6.4:1 | 0.93:1 |

In contrast to the parent enzyme the mutation D557E reduces the influence of 6-gal-lac on lactose measurement dramatically. The ratio lactose:6-gal-lac more than doubles, while the ratio lactose:glucose stays almost identical. The ratio lactose:lactose+6-gal-lac shows that there is only a small influence of the signal strength when 6-gal-lac is added to a lactose solution. Therefore, the enzyme variant is an extraordinarily improved biocatalyst for the measurement of lactose in the presence of the major interfering sugars in milk - glucose and galacto-oligosaccharides.

### References

Canevascini et al., 1991, Eur. J. Biochem. 198: 43-52
Gao et al. (2001) Plos Genetics, 7 (1): e1001264
Harreither et al. (2011) Appl. Environ. Microbiol. 77:1804-1815.
Ludwig et al. (2010) Chem. Phys. Chem. 11:2674-2697
Safina et al. (2010) Electrochimica Acta 55: 7690-7695.
Sako et al. (1999) Int. Dairy J., 9: 69-80
Tasca et al. (2010) Bioelect. 25:1710-1716.
Tasca et al. (2010b) Bioelect. 25:1710-1716.
Tasca et al. (2011) Anal. Chem. 83:3042-3049.
Tasca et al. (2011b). Analyst 136:2033-2036.
Weis R, et al. (2004). FEMS Yeast Res. 5:179 -189.

## Claims

1. A modified cellobiose dehydrogenase (CDH) or its functional flavodehydrogenase domain comprising a modified flavodehydrogenase domain based on one of the unmodified flavodehydrogenase domains according to amino acids 253-829 of SEQ ID NO: 4, amino acids 253-831 of SEQ ID NO: 1, amino acids 269-845 of SEQ ID NO: 2, amino acids 249-787 of SEQ ID NO: 3, amino acids 251-828 of SEQ ID NO: 5, amino acids 251-829 of SEQ ID NO: 6, amino acids 246-785 of SEQ ID NO: 17; said modified flavodehydrogenase domain has a sequence with at least 75% sequence identity with one of said unmodified flavodehydrogenase domains and further comprises a substitution mutation at least at one of the amino acids of the galacto-oligosaccharide binding motif corresponding to amino acids 555-557 and 639-641 of SEQ ID NO: 4 (CDH from N. *crassa*), wherein the ratio of lactose oxidation activity to the oxidation activity of 6-galactosyllactose is at least 2:1.

2. The modified CDH or its functional flavodehydrogenase domain according to claim 1, further comprising a substitution at the amino acid corresponding to N721 of SEQ ID NO: 5 (CDH from M. *thermophilum*), preferably by glutamine, isoleucine, threonine or leucine or a substitution of the asparagine in the active center motif having the amino acid sequence NHW, preferably by glutamine, isoleucine, threonine or leucine.

3. The modified CDH or its functional flavodehydrogenase domain according to claim 2, wherein active center motif has the sequence X₁X₂NHWX₃X₄X₅, wherein X₁ is any amino acid, preferably selected from N, C and R; X₂ is selected from S and A; X₃ is selected from V, M and I; X₄ is any amino acid, preferably selected from G and S; and X₅ is any amino acid, preferably selected from S, A and T; especially preferred X₄ and X₅ are not both S.

4. The modified CDH or its functional flavodehydrogenase domain according to any one of claims 1 to 3, **characterised in that** the CDH is of *dikaryota*, preferably selected from *ascomycota*, preferably wherein the CDH is a modified CDH of a CDH from *Myriococcum thermophilum, Corynascus thermophilus, Chaetomium atrobrunneum (Myceliophthora fergusii), Hypoxylon haematostroma, Neurospora crassa, Stachybotrys bisby* or *Humicola insolens.*

5. The modified CDH or its functional flavodehydrogenase domain according to any one of claims 1 to 4, comprising a modified flavodehydrogenase domain based on one of the unmodified flavodehydrogenase domains according to amino acids 253-831 of SEQ ID NO: 1, amino acids 269-845 of SEQ ID NO: 2, amino acids 249-787 of SEQ ID NO: 3, amino acids 253-829 of SEQ ID NO: 4, amino acids 251-828 of SEQ ID NO: 5, amino acids 251-829 of SEQ ID NO: 6, amino acids 246-785 of SEQ ID NO: 17; said modified flavodehydrogenase domain has a sequence with at least 80%, preferably at least 90%, at least 95%, at least 98%, in particular preferred at least 99%, sequence identity with one of said unmodified flavodehydrogenase domains and further comprises the substitution mutation at least at an amino acid corresponding to amino acids 555-557 and 639-641 of SEQ ID NO: 4.

6. The modified CDH or its functional flavodehydrogenase domain according to any one of claims 1 to 5, wherein the amino acids of the galacto-oligosaccharide binding motif correspond to amino acids 555-557 and 639-641 of SEQ ID NO: 4 is at amino acids 556-558 and 640-642 of SEQ ID NO: 1, amino acids 571-573 and 655-657 of SEQ ID NO: 2, amino acids 550-552 and 635-637 of SEQ ID NO: 3, amino acids 554-556 and 638-640 of SEQ ID NO: 5, amino acids 554-556 and 638-640 of SEQ ID NO: 6, amino acids 548-550 and 633-635 of SEQ ID NO: 17.

7. The modified CDH or its functional flavodehydrogenase domain according to any one of claims 1 to 6, being recombinantly produced by *Pichia pastoris,* isolated by diafiltration, ion exchange chromatography and preferably being further purified by hydrophobic interaction chromatography.

8. The modified CDH or its functional flavodehydrogenase domain according to any one of claims 1 to 7, wherein the substitution is at an amino acid corresponding to N555, D557 and S639 of the CDH of SEQ ID NO: 4.

9. The modified CDH or its functional flavodehydrogenase domain according to claim 8, wherein the substitution is by an amino acid corresponding to a substitution selected from D557V, D557L, S639V, S639N, N555M, S639I, D557R, S639G, D557T, D557I, D557E, N555L, D557P, S639M, S639R, D557G, S639E, D557A, S639Q, S639K, S639C, D557S, S639D, D557M, D557H, S639W, D557W, S639F, N555D, D557Q, S639Y, S639L, N555K, S639H, D557C of the CDH of SEQ ID NO: 4.

10. The modified CDH or its functional flavodehydrogenase domain according to any one of claims 1 to 9, wherein the ratio of lactose oxidation activity to the oxidation activity of 6-galactosyllactose is at least 3:1.

11. A nucleic acid molecule encoding a modified CDH or its functional flavodehydrogenase domain according to any one of claims 1 to 10.

12. A method of producing a modified CDH or its functional flavodehydrogenase domain according to any one of claims 1 to 10, comprising recombinantly expressing a nucleic acid molecule according to claim 11 in a host cell.

13. An electrode comprising an immobilised cellobiose dehydrogenase or its functional flavodehydrogenase domain according to any one of claims 1 to 10;
preferably wherein the cellobiose dehydrogenase is immobilised by adsorption, complex formation, especially preferred via an additional complexing linker, covalent or ionic linkage, and/or wherein preferably the immobilized cellobiose dehydrogenase is cross-linked, in particular by bifunctional agents, to increase stability or activity.

14. Method of detecting or quantifying lactose in a sample comprising the step of oxidizing lactose in said sample with a modified CDH or its functional flavodehydrogenase domain according to any one of claims 1 to 10, or an electrode according to claim 13 and detecting or quantifying said oxidation, preferably wherein said sample comprises or is suspected of comprising a galacto-oligosaccharide, especially preferred a milk or milk product containing sample.

15. A lactose assay kit comprising the modified Cellobiose dehydrogenase or its functional flavodehydrogenase domain according to any of the claims 1 to 10 or an electrode according to claim 13 and a sample holding means and/or lactose standards.

## Patentansprüche

1. Modifizierte Cellobiose-Dehydrogenase (CDH) oder ihre funktionale Flavodehydrogenase-Domäne, umfassend eine modifizierte Flavodehydrogenase-Domäne auf Basis einer der unmodifizierten Flavodehydrogenase-Domänen gemäß den Aminosäuren 253-829 von SEQ ID NO: 4, Aminosäuren 253-831 von SEQ ID NO: 1, Aminosäuren 269-845 von SEQ ID NO: 2, Aminosäuren 249-787 von SEQ ID NO: 3, Aminosäuren 251-828 von SEQ ID NO: 5, Aminosäuren 251-829 von SEQ ID NO: 6, Aminosäuren 246-785 von SEQ ID NO: 17; wobei die modifizierte Flavodehydrogenase-Domäne eine Sequenz mit zumindest 75 % Sequenzidentität mit einer der unmodifizierten Flavodehydrogenase-Domänen aufweist und ferner eine Substitutions-Mutation zumindest an einer der Aminosäuren des Galacto-Oligosaccharid-Bindungsmotivs entsprechend den Aminosäuren 555-557 und 639-641 von SEQ ID NO: 4 (CDH von *N. crassa)* umfasst, wobei das Verhältnis von Lactose-Oxidations-Aktivität zu der Oxidations-Aktivität von 6-Galactosyllactose zumindest 2:1 beträgt.

2. Modifizierte CDH oder ihre funktionale Flavodehydrogenase-Domäne nach Anspruch 1, ferner umfassend eine Substitution an der Aminosäure entsprechend N721 von SEQ ID NO: 5 (CDH von M. *thermophilum),* vorzugsweise durch Glutamin, Isoleucin, Threonin oder Leucin, oder eine Substitution des Asparagins in dem aktiven Zentralmotiv, das die Aminosäuresequenz NHW aufweist, vorzugsweise durch Glutamin, Isoleucin, Threonin oder Leucin.

3. Modifizierte CDH oder ihre funktionale Flavodehydrogenase-Domäne nach Anspruch 2, wobei das aktive Zentralmotiv die Sequenz X₁X₂NHWX₃X₄X₅ aufweist, wobei X₁ eine beliebige Aminosäure ist, vorzugsweise ausgewählt aus N, C und R; X₂ aus S und A ausgewählt ist; X₃ aus V, M und I ausgewählt ist; X₄ eine beliebige Aminosäure ist, vorzugsweise ausgewählt aus G und S; und X₅ eine beliebige Aminosäure ist, vorzugsweise ausgewählt aus S, A und T; besonders bevorzugt X₄ und X₅ nicht beide S sind.

4. Modifizierte CDH oder ihre funktionale Flavodehydrogenase-Domäne nach einem der Ansprüche 1 bis 3, **dadurch gekennzeichnet, dass** die CDH von *dikaryota* stammt, vorzugsweise ausgewählt aus *ascomycota,* wobei vorzugsweise die CDH eine modifizierte CDH einer CDH von *Myriococcum thermophilum, Corynascus thermophilus, Chaetomium atrobrunneum (Myceliophthora fergusii), Hypoxylon haematostroma, Neurospora crassa, Stachybotrys bisby* oder *Humicola insolens* ist.

5. Modifizierte CDH oder ihre funktionale Flavodehydrogenase-Domäne nach einem der Ansprüche 1 bis 4, umfassend eine modifizierte Flavodehydrogenase-Domäne auf Basis einer der unmodifizierten Flavodehydrogenase-Domänen gemäß den Aminosäuren 253-831 von SEQ ID NO: 1, Aminosäuren 269-845 von SEQ ID NO: 2, Aminosäuren 249-787 von SEQ ID NO: 3, Aminosäuren 253-829 von SEQ ID NO: 4, Aminosäuren 251-828 von SEQ ID NO: 5, Aminosäuren 251-829 von SEQ ID NO: 6, Aminosäuren 246-785 von SEQ ID NO: 17; wobei die modifizierte Flavodehydrogenase-Domäne eine Sequenz mit zumindest 80 %, vorzugsweise zumindest 90 %, zumindest 95 %, zumindest 98 %, insbesondere bevorzugt zumindest 99 %, Sequenzidentität mit einer der unmodifizierten Flavodehydrogenase-Domänen aufweist und ferner die Substitutions-Mutation zumindest an einer Aminosäure entsprechend den Aminosäuren 555-557 und 639-641 von SEQ ID NO: 4 umfasst.

6. Modifizierte CDH oder ihre funktionale Flavodehydrogenase-Domäne nach einem der Ansprüche 1 bis 5, wobei die Aminosäuren des Galacto-Oligosaccharid-Bindungsmotivs entsprechend den Aminosäuren 555-557 und 639-641 von SEQ ID NO: 4 an den Aminosäuren 556-558 und 640-642 von SEQ ID NO: 1, an den Aminosäuren 571-573 und 655-657 von SEQ ID NO: 2, an den Aminosäuren 550-552 und 635-637 von SEQ ID NO: 3, an den Aminosäuren 554-556 und 638-640 von SEQ ID NO: 5, an den Aminosäuren 554-556 und 638-640 von SEQ ID NO: 6, an den Aminosäuren 548-550 und 633-635 von SEQ ID NO: 17 vorliegen.

7. Modifizierte CDH oder ihre funktionale Flavodehydrogenase-Domäne nach einem der Ansprüche 1 bis 6, die rekombinant von *Pichia pastoris* produziert wird, durch Diafiltration, Ionenaustausch-Chromatographie isoliert und vorzugsweise durch hydrophobe Interaktions-Chromatographie weiter aufgereinigt wird.

8. Modifizierte CDH oder ihre funktionale Flavodehydrogenase-Domäne nach einem der Ansprüche 1 bis 7, wobei die Substitution an einer Aminosäure entsprechend N555, D557 und S639 der CDH von SEQ ID NO: 4 vorliegt.

9. Modifizierte CDH oder ihre funktionale Flavodehydrogenase-Domäne nach Anspruch 8, wobei die Substitution durch eine Aminosäure entsprechend einer Substitution ausgewählt aus D557V, D557L, S639V, S639N, N555M, S639I, D557R, S639G, D557T, D557I, D557E, N555L, D557P, 5639M, S639R, D557G, S639E, D557A, S639Q, S639K, S639C, D557S, S639D, D557M, D557H, S639W, D557W, S639F, N555D, D557Q, S639Y, S639L, N555K, S639H, D557C der CDH von SEQ ID NO: 4 erfolgt.

10. Modifizierte CDH oder ihre funktionale Flavodehydrogenase-Domäne nach einem der Ansprüche 1 bis 9, wobei das Verhältnis der Lactose-Oxidations-Aktivität zu der Oxidations-Aktivität von 6-Galactosyllactose zumindest 3:1 beträgt.

11. Nukleinsäuremolekül, das für eine modifizierte CDH oder ihre funktionale Flavodehydrogenase-Domäne nach einem der Ansprüche 1 bis 10 kodiert.

12. Verfahren zur Herstellung einer modifizierten CDH oder ihrer funktionalen Flavodehydrogenase-Domäne nach einem der Ansprüche 1 bis 10, umfassend das rekombinante Exprimieren eines Nukleinsäuremoleküls nach Anspruch 11 in einer Wirtszelle.

13. Elektrode, umfassend eine immobilisierte Cellobiose-Dehydrogenase oder ihre funktionale Flavodehydrogenase-Domäne nach einem der Ansprüche 1 bis 10;
wobei vorzugsweise die Cellobiose-Dehydrogenase durch Adsorption, Komplexbildung, besonders bevorzugt über einen zusätzlichen Komplexierungslinker, kovalente oder Ionenbindung immobilisiert ist, und/oder wobei vorzugsweise die immobilisierte Cellobiose-Dehydrogenase insbesondere durch bifunktionale Agenzien vernetzt ist, um die Stabilität oder Aktivität zu erhöhen.

14. Verfahren zum Nachweisen oder Quantifizieren von Lactose in einer Probe, umfassend den Schritt des Oxidierens von Lactose in der Probe mit einer modifizierten CDH oder ihrer funktionalen Flavodehydrogenase-Domäne nach einem der Ansprüche 1 bis 10, oder einer Elektrode nach Anspruch 13, und Nachweisen oder Quantifizieren der Oxidation, wobei vorzugsweise die Probe ein Galacto-Oligosaccharid umfasst oder vermutlich umfasst, besonders bevorzugt eine Milch oder ein Milchprodukt enthaltende Probe.

15. Lactose-Assay-Kit, umfassend die modifizierte Cellobiose-Dehydrogenase oder ihre funktionale Flavodehydrogenase-Domäne nach einem der Ansprüche 1 bis 10 oder eine Elektrode nach Anspruch 13 und ein Probenaufnahmemittel und/oder Lactose-Standards.

## Revendications

1. Cellobiose déshydrogénase (CDH) modifiée ou son domaine flavodéshydrogénase modifié comprenant un domaine flavodéshydrogénase modifié basé sur l'un des domaines flavodéshydrogénase non modifiés conformément aux acides aminés 253 à 829 de SEQ ID N°: 4, aux acides aminés 253 à 831 de SEQ ID N°: 1, aux acides aminés 269 à 845 de SEQ ID N°: 2, aux acides aminés 249 à 787 de SEQ ID N°: 3, aux acides aminés 251 à 828 de SEQ ID N°: 5, aux acides aminés 251 à 829 de SEQ ID N°: 6, aux acides aminés 246 à 785 de SEQ ID N°: 17 ; ledit domaine flavodéshydrogénase modifié présente une séquence avec une identité de séquence d'au moins 75 % avec l'un desdits domaines flavodéshydrogénase non modifiés et comprend en outre une mutation par substitution au moins au niveau de l'un des acides aminés du motif de liaison galacto-oligosaccharide correspondant aux acides aminés 555 à 557 et 639 à 641 de SEQ ID N° : 4 (CDH provenant de *N. crassa),* dans laquelle le rapport de l'activité d'oxydation du lactose à l'activité d'oxydation du 6-galactosyllactose vaut au moins 2:1.

2. CDH modifiée ou son domaine flavodéshydrogénase fonctionnel selon la revendication 1, comprenant en outre une substitution au niveau de l'acide aminé correspondant à N721 de SEQ ID N° : 5 (CDH provenant de M. *thermophilum),* de préférence par la glutamine, l'isoleucine, la thréonine ou la leucine ou une substitution de l'asparagine dans le motif central actif présentant la séquence d'acides aminés NHW, de préférence par la glutamine, l'isoleucine, la thréonine ou la leucine.

3. CDH modifiée ou son domaine flavodéshydrogénase fonctionnel selon la revendication 2, ledit motif central actif présentant la séquence X₁X₂NHWX₃X₄X₅, dans laquelle X₁ représente un quelconque acide aminé, de préférence choisi parmi N, C et R ; X₂ est choisi parmi S et A ; X₃ est choisi parmi V, M et I ; X₄ représente un quelconque acide aminé, de préférence choisi parmi G et S ; et X₅ représente un quelconque acide aminé, de préférence choisi parmi S, A et T ; sont particulièrement préférés X₄ et X₅ non tous deux S.

4. CDH modifiée ou son domaine flavodéshydrogénase fonctionnel selon l'une quelconque des revendications 1 à 3, **caractérisé en ce que** la CDH provient de *dicaryotes,* de préférence choisis parmi les *ascomycètes,* de préférence dans lesquels la CDH est une CDH modifiée d'une CDH provenant de *Myriococcum thermophilum, Corynascus thermophilus, Chaetomium atrobrinneum (Myceliophthora fergusii), Hypoxylon haematostroma, Neurospora crassa, Stachybotrys bisby ou Humicola insolens.*

5. CDH modifiée ou son domaine flavodéshydrogénase fonctionnel selon l'une quelconque des revendications 1 à 4, comprenant un domaine flavodéshydrogénase modifié basé sur l'un des domaines flavodéshydrogénase non modifiés conformément aux acides aminés 253 à 831 de SEQ ID N°: 1, aux acides aminés 269 à 845 de SEQ ID N°: 2, aux acides aminés 249 à 787 de SEQ ID N°: 3, aux acides aminés 253 à 829 de SEQ ID N°: 4, aux acides aminés 251 à 828 de SEQ ID N°: 5, aux acides aminés 251 à 829 de SEQ ID N°: 6, aux acides aminés 246 à 785 de SEQ ID N°: 17 ; ledit domaine flavodéshydrogénase modifié présente une séquence avec une identité de séquence d'au moins 80 %, de préférence d'au moins 90 %, d'au moins 95 %, d'au moins 98 %, en particulier préférée d'au moins 99 %, avec l'un desdits domaines flavodéshydrogénase non modifiés et comprend en outre la mutation par substitution au moins au niveau d'un acide aminé correspondant aux acides aminés 555 à 557 et 639 à 641 de SEQ ID N° : 4.

6. CDH modifiée ou son domaine flavodéshydrogénase fonctionnel selon l'une quelconque des revendications 1 à 5, dans lequel les acides aminés du motif de liaison du galacto-oligosaccharide correspondent aux acides aminés 555 à 557 et 639 à 641 de SEQ ID N° : 4 est au niveau des acides aminés 556 à 558 et 640 à 642 de SEQ ID N° : 1, des acides aminés 571 à 573 et 655 à 657 de SEQ ID N° : 2, des acides aminés 550 à 552 et 635 à 637 de SEQ ID N° : 3, des acides aminés 554 à 556 et 638 à 640 de SEQ ID N° : 5, des acides aminés 554 à 556 et 638 à 640 de SEQ ID N° : 6, des acides aminés 548 à 550 et 633 à 635 de SEQ ID N° : 17.

7. CDH modifiée ou son domaine flavodéshydrogénase fonctionnel selon l'une quelconque des revendications 1 à 6, étant produit de manière recombinante par *Pichia pastoris,* isolé par diafiltration, chromatographie par échange d'ions et de préférence étant en outre purifié par chromatographie par interaction hydrophobe.

8. CDH modifiée ou son domaine flavodéshydrogénase fonctionnel selon l'une quelconque des revendications 1 à 7, dans lequel la substitution est au niveau d'un acide aminé correspondant à N555, D557 et S639 de la CDH de SEQ ID N° : 4.

9. CDH modifiée ou son domaine flavodéshydrogénase fonctionnel selon la revendication 8, dans lequel la substitution est par un acide aminé correspondant à une substitution choisie parmi D557V, D557L, D639V, S639N, N555M, S339I, S557R, S639G, D557T, D557I, D557E, N555L, D557P, S639M, S639R, D557G, S639E, D557A, S639Q, S639K, S639C, D557S, S639D, D557M, D557H, S639W, D557W, S639F, N555D, D557Q, S639Y, S639L, N555K, S639H, D557C de la CDH de SEQ ID N° : 4.

10. CDH modifiée ou son domaine flavodéshydrogénase fonctionnel selon l'une quelconque des revendications 1 à 9, dans lequel le rapport de l'activité d'oxydation du lactose à l'activité d'oxydation du 6-galactosyllactose vaut au moins 3:1.

11. Molécule d'acide nucléique codant une CDH modifiée ou son domaine flavodéshydrogénase fonctionnel selon l'une quelconque des revendications 1 à 10.

12. Procédé de production d'une CDH modifiée ou de son domaine flavodéshydrogénase fonctionnel selon l'une quelconque des revendications 1 à 10, comprenant l'expression recombinante d'une molécule d'acide nucléique selon la revendication 11 dans une cellule hôte.

13. Electrode comprenant une cellobiose déshydrogénase immobilisée ou son domaine flavodéshydrogénase fonctionnel selon l'une quelconque des revendications 1 à 10 ;
de préférence dans laquelle la cellobiose déshydrogénase est immobilisée par adsorption, formation de complexe, particulièrement préférée via un lieur de complexation supplémentaire, liaison covalente ou ionique, et/ou dans laquelle de préférence la cellobiose déshydrogénase immobilisée est réticulée, en particulier par des agents bifonctionnels, pour augmenter la stabilité ou l'activité.

14. Procédé de détection ou de quantification du lactose dans un échantillon comprenant l'étape d'oxydation du lactose dans ledit échantillon avec une CDH modifiée ou son domaine flavodéshydrogénase fonctionnel selon l'une quelconque des revendications 1 à 10, ou une électrode selon la revendication 13 et de détection ou de quantification de ladite oxydation, de préférence dans lequel ledit échantillon comprend ou est susceptible de comprendre un galacto-oligosaccharide, est particulièrement préférée un échantillon contenant du lait ou un produit laitier.

15. Kit de dosage du lactose comprenant la cellobiose déshydrogénase modifiée ou son domaine flavodéshydrogénase fonctionnel selon l'une quelconque des revendications 1 à 10 ou une électrode selon la revendication 13 et un moyen support d'échantillon et/ou des étalons de lactose.
